# EUROPEAN PATENT APPLICATION

(11) **EP 3 501 697 A1**
(43) Date of publication of application: **26.06.2019**
(21) Application number: 18207850.1
(22) Date of filing: 22.11.2018
(51) Int. Cl.: B22F 3/105, B22F 3/15, B22F 3/24, B22F 7/06, B22F 7/08, F16K 1/00, B21C 37/06, A61F 2/32, B33Y 80/00, B33Y 10/00, B22F 3/12

(54) **A MANUFACTURING METHOD**

(30) Priority: 22.12.2017 GB 201721769
(71) Applicant: Rolls-Royce Power Engineering PLC, Derby DE24 8BJ (GB)
(72) Inventor: Brown, Richard, Derby, Derbyshire DE24 8BJ (GB)
(74) Representative: Rolls-Royce plc

(57) **Abstract**

There is provided a method of manufacture of a component from multiple materials, and a consolidated component formed thereby. The method includes the steps of forming a component body comprising a first material, the component body defining a cavity, filling at least a portion of the cavity with a second material, and performing hot isostatic pressing of the component body and second material to form a consolidated component. The process offers a reduced-complexity, reduced-part-count method of manufacture for components formed of multiple materials.

## Description

### Field of the Disclosure

The present disclosure relates to a method of manufacturing a component formed of multiple materials. More specifically, it relates to a method of manufacturing a component formed of multiple materials using hot isostatic pressing.

### Background

Hot isostatic pressing (HIP) is a well-known manufacturing or processing technique in which high isostatic pressure is applied to a material, typically a powder within a canister, at an elevated temperature to provide isostatic compression. Typically, this process is performed in an inert atmosphere, e.g. in an argon gas atmosphere. This has applications in reducing the porosity and increasing density of metal and ceramic materials, or in forming a compact solid from a powder body in sintering processes.

Hot isostatic pressing is typically used in the manufacture of a wide range of components. For the production of components formed of multiple materials, the manufacturing process will generally involve multiple machining and hot isostatic pressing stages. This can result in a complex manufacturing process. Furthermore, where a manufacturing process includes multiple hot isostatic pressing stages, e.g. where separate initial component parts undergo a first HIP cycle for densification and are subsequently bonded together using a second HIP cycle, this repeated exposure of component parts to high temperatures can cause undesirable degradation in the mechanical properties of the component parts due to grain coarsening resulting from multiple cycles at elevated temperatures.

An example of a known process in which these problems typically occur includes the manufacture of valves having hard-facings, which typically require a minimum of two HIP cycles: an initial HIP cycle to form and densify the main body of the valve, and a second HIP 'bonding' cycle to bond the hard-facing component part to the main body of the valve. However, such problems may also be prevalent in many different technical fields where it is desired to produce components formed of multiple materials using a process of hot isostatic pressing.

Accordingly, there is a desire to provide a method of manufacture of a component formed of multiple materials which reduces, preferably overcomes, the above mentioned problems.

### Summary

In a first aspect, there is provided a method of manufacturing a component from multiple materials including the steps of:
forming a component body comprising a first material, the component body defining a cavity;
filling at least a portion of the cavity with a second material; and
performing hot isostatic pressing of the component body and second material to form a consolidated component.

During the step of hot isostatic pressing, the second material located in the cavity defined by the component body undergoes densification and/or is consolidated into a solid mass whilst undergoing bonding to the component body, thus forming a consolidated component comprising the first material of the component body and the second material.

In this way, the present process may allow for a reduction in lead time and supply chain complexity which is at least in part enabled by the reduction in the total number of HIP stages needed to form a consolidated component from multiple materials. The present process may require only a single HIP cycle to form a consolidated component.

Furthermore, the present process may also allow for the total part count of the manufacturing process to be reduced relative to some known manufacturing processes. This is because in comparison to some known processes, where a first material component and a second material component may be joined together using HIP to form a consolidated component, in the present process, the second material component does not exist as a separate component before the HIP step. Rather, the second material portion of the component is added to the main component body from raw material.

Optional features will now be set out. These are applicable singly or in any combination with any aspect.

The terms "first material" and "second material" are generally used herein to indicate materials which differ from one another. That is, the first material and the second material may differ in class of material (e.g. metal/ceramic/polymer), or may both be the same class of material but having different chemical compositions (e.g. two metal alloys with different compositions) or different physical properties e.g. different densities, particle sizes etc. The precise materials of the component body and the second material are not particularly limited. Indeed, it is contemplated that the present method may be suitable for use with a wide range of material combinations, as will be readily understood by the skilled person.

The second material may be a powder. A powder material may be advantageous as it may be easier to completely fill a cavity having complex geometry with a powder material.

The method may comprise forming the component body using an additive manufacturing process. For example, the method may comprise forming the component body using any one of laser/electron beam melting/sintering; powder bed fusion; 3D printing; selective laser sintering/melting; a blown powder process; direct laser deposition; freeform manufacture; or additive layer manufacturing. The skilled person will be aware of a wide range additive manufacturing processes which would be suitable for use in the manufacturing process disclosed herein.

Use of such an additive manufacturing process may enable the production of complex part geometry. For example, additive manufacture may allow more simple production of parts having re-entrant portions, or fine detail.

Alternatively, the method may comprise forming the component body by machining. In particular, machining may be particularly advantageous for simple components, due to the typically lower manufacturing costs in comparison to additive manufacturing.

The method may comprise forming the component body as a net or near-net shape component. As is generally understood, the term "net shape" herein refers to a component body that requires no post-HIP re-shaping or machining to satisfy its final design shape criteria. The term "near net shape" is a shape that requires only minimal machining or reshaping post-HIP to meet its final design shape criteria.

The method may comprise forming the component body comprising one or more sacrificial portions which may partly define the cavity. The method may further comprise removing the one or more sacrificial portions after performing hot isostatic pressing of the component body and second material to form a consolidated component.

Alternatively, the method may comprise forming the component body such that the cavity is entirely defined by non-sacrificial portions of the component body. Accordingly, the component body may be a net shape component.

The geometry of the cavity may be selected so as to control the deformation which occurs during the HIP process. For example, where the cavity is partially defined by a sacrificial portion of the component body, the cavity geometry may be selected so that only deformation of the sacrificial portion of the component body occurs during HIP, or so that only a limited amount of deformation occurs to non-sacrificial portions of the component body. This may be done, for example, by controlling the wall thickness of the sacrificial material to encourage preferential deformation of the sacrificial material over deformation of non-sacrificial portions of the component body. In this way, unwanted deformation of the body of the consolidated component may be reduced or avoided.

The method will generally comprise filling at least a portion of the cavity with the second material through at least one, and optionally multiple, external openings. However, alternative embodiments are also contemplated. For example, one alternative embodiment includes filling a half-formed cavity mid-way through the process of forming a component body by, for example, additive manufacturing. In such an embodiment, the component body defines a cavity filled with a second material where the cavity does not have any external openings. Here the term 'filling' includes both part filling, and totally filling the cavity. The total volume of second material inserted into the cavity may therefore be equal to or less than the total volume of the cavity to be filled. The precise amount of second material may be selected as appropriate for the intended design of the consolidated component.

Whilst the above description makes reference to 'a cavity' it will clearly be understood that the process is not limited to a single cavity. The method may comprise forming the component body to define more than one cavity. For example, the method may comprise forming component body to define two, three, or four or more cavities. The number of cavities is not particularly limited and may be selected as appropriate given the desired features of the consolidated component. Where there are multiple cavities, the method may comprise at least part-filling all cavities with a second material before the step of hot isostatic pressing to form the consolidated component. It will be understood that multiple different second materials may be used to fill the respective multiple cavities before the hot isostatic pressing step. Furthermore, where the method includes forming a component body defining multiple cavities, such cavities may be of different shapes and sizes and may be filled to differing degrees, as appropriate.

In a second aspect, there is provided a consolidated component formed by the method according to the first aspect.

As discussed above, the present process requires only a single HIP cycle to achieve both densification of the component, and bonding of the first and second materials. In this case, the overall mechanical properties of the component may be improved, because it is not necessary for the component body to undergo multiple HIP cycles at elevated temperatures which may cause grain coarsening.

It is contemplated that a wide range of components across a broad range of technical fields could be formed using the method of manufacture disclosed herein. Indeed, the present method may be suitable for any components where multiple materials are required, and when hot isostatic pressing is a generally preferred manufacturing process. Examples components include but are in no way limited to: valves, components having hard facings, cladded components, components having embedded electrically conductive material etc.

Such components may find use in technical fields including but not limited to nuclear engineering, submarines, oil & gas engineering, marine engineering, aerospace engineering, automotive engineering, space exploration, and the medical field.

### Brief Description of the Drawings

Embodiments will now be described by way of example with reference to the accompanying drawings in which:
Figures 1(a)-(d) show selected schematic sections taken throughout a method of manufacture of a valve with hard facing;
Figures 2(a)-(d) show selected schematic sections taken throughout a method of manufacture of a pipe with internal cladding;
Figures 3(a)-(d) show selected schematic sections taken throughout a method of manufacture of an artificial hip joint having a hard-wearing coating material; and
Figures 4(a)-(c) show 3D rendered versions of the articles shown in Figure 3.

### Detailed Description and Further Optional Features

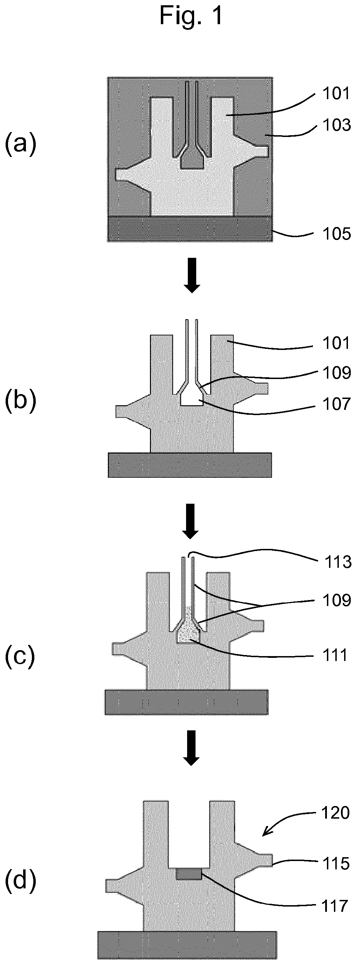

Figure 1 shows selected schematic sections taken throughout a method of manufacture of a valve with hard facing.

Figure 1(a) shows the step of forming the component body 101, here a valve body using an additive manufacturing process of laser melting/sintering. In a manner well-known to those in the art, a powder bed 103 is placed on a supporting baseplate 105, and selected areas of the powder bed as defined by a 3D product model are melted using a laser to form a solid component body. After the additive manufacture process is complete, the solid component body is surrounded by un-melted powder which is subsequently removed once the melting/sintering process is complete to leave the component body as shown in Figure 1(b). The powder is typically vacuumed away, however where the internal features are complex, the component may be manipulated to encourage the powder to flow out. For very complex parts, ultrasonic wash stations may be used. The component body is here made entirely from grade 316 stainless steel.

The component body 101 is formed to define a cavity 107. Here, the component body 101 is formed to be a near-net shape component comprising sacrificial portions 109. In the present example, the cavity in the component body is partially defined by these sacrificial portions of the component body, and partially defined by non-sacrificial portions. The shape of the cavity is selected so as to control deformation during the hot isostatic pressing step.

In Figure 1(c), the cavity 107 is part-filled with a powder 111 of a second material (here a material from the Tristelle alloy family) via an opening 113. The level of filling is selected as appropriate given the desired final design of the component. Here, because the incorporation of the second material into the component is for the purpose of producing a hard-facing on a valve, it is not necessary to fill the entire cavity. Rather, by part-filling the cavity, the process can achieve a more economical usage of raw materials.

The entire component then undergoes a hot isostatic pressing cycle, during which the second material powder 111 densifies and bonds to the component body 101 at the cavity walls, thereby forming a consolidated component 120.

Post-densification, the consolidated component is further processed (e.g. machined) to remove sacrificial portions 109 of the component body, along with excess second material. The final product, shown in Figure 1(d) is a consolidated component comprising a valve body portion 115 of a first material having an integrated hard facing 117 of a second material at the location of the original cavity.

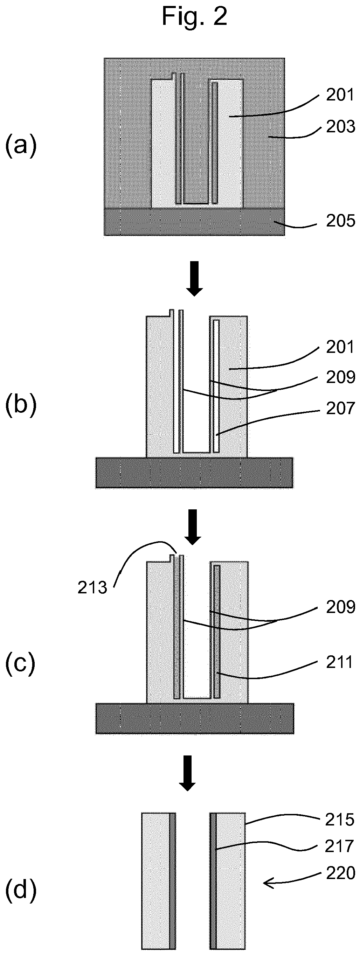

Figure 2 shows selected schematic sections taken throughout a method of manufacture of a pipe with internal cladding. The method of manufacture is similar to that described above in relation to manufacture of a valve with hard facing. Initially, a component body 201 of a first material (e.g. mild steel) is fabricated using a method of additive manufacture from a powder bed 203 supported by a baseplate 205. Here, the component body 201 is formed to define an annular cavity 207 around the central shaft of the pipe. The cavity has an opening 213 through which a powder 211 of a second material (e.g. stainless steel) is inserted to fill the cavity. The component body is formed such that the cavity 207 is partially defined by sacrificial portions 209 of the component body (here the radially inner cavity wall), and partially defined by non-sacrificial portions (here the radially outer cavity wall).

In the same manner as discussed in relation to the previous example, once the cavity 207 has been filled with powder 211 the component undergoes a hot isostatic pressing cycle to form the consolidated component 220 followed by removal of sacrificial portions of the component body. The final product is a consolidated component comprising a pipe body portion 215 of a first material having exposed integrated interior surface cladding 217 of a second material at the location of the original cavity.

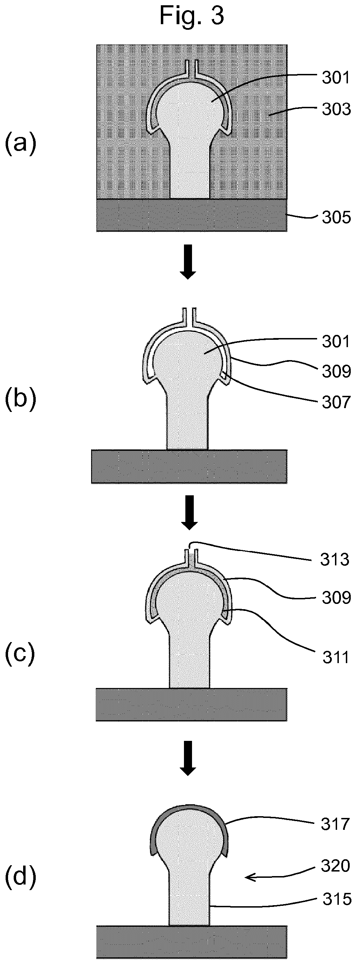

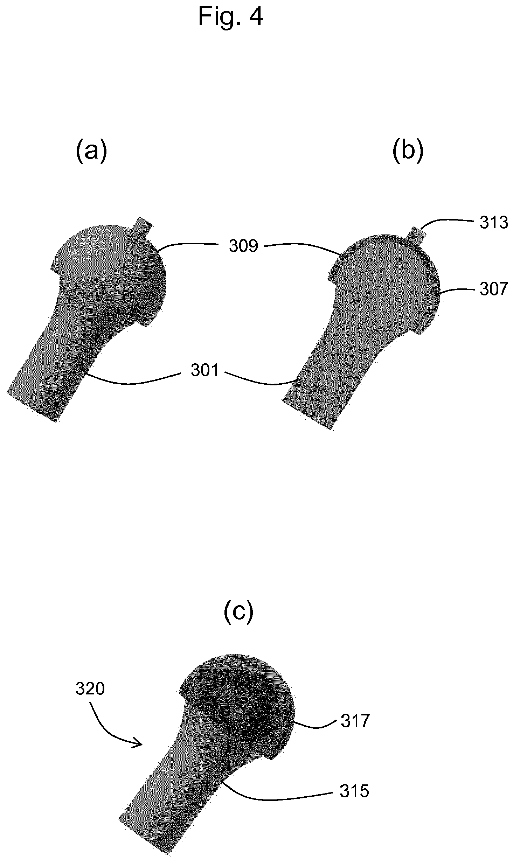

Figures 3 and 4 show selected schematic sections taken throughout a method of manufacture of an artificial hip joint having a hard-wearing coating material covering a part of the joint. Figure 4 (a) and (b) are 3D rendered equivalents of a component as shown in Figure 3 (b). Figure 4(c) is a 3D rendered equivalent of Fig. 3(d). The method of manufacture is similar to that described above in relation to manufacture of a valve with hard facing. Initially, a component body 301 of a first material is fabricated using a method of additive manufacture from a powder bed 303 supported by a baseplate 305. Here, the component body 301 is formed to define an approximately hemispherical cavity 307 surrounding the ball joint portion of the artificial hip joint. An opening 313 is provided at the centre of the hemispherical cavity, and the cavity is filled with powder 311 of a second material through said opening. The component body is formed such that the hemispherical cavity 307 is partially defined by a radially outer shell which is a sacrificial portion 309 of the component body, and partially defined by a non-sacrificial ball-joint portion of the component body.

In the same manner as discussed in relation to the previous example, the component undergoes a hot isostatic pressing cycle to form the consolidated component 320 followed by removal of sacrificial portions of the component body. The final product is a consolidated component comprising a hip joint body portion 315 of a first material having an integrated surface covering 317 of a second material at the location of the original cavity.

It will be understood that the disclosure is not limited to the embodiments above-described and various modifications and improvements can be made without departing from the concepts described herein. Except where mutually exclusive, any of the features may be employed separately or in combination with any other features and the disclosure extends to and includes all combinations and sub-combinations of one or more features described herein.

## Claims

1. A method of manufacturing a component from multiple materials including the steps of:
forming a component body comprising a first material, the component body defining a cavity;
filling at least a portion of the cavity with a second material; and
performing hot isostatic pressing of the component body and second material to form a consolidated component.

2. The method of manufacturing according to Claim 1 wherein the second material is a powder material.

3. The method according to Claim 1 or Claim 2 comprising forming the component body using an additive manufacturing process.

4. The method according to Claim 3 wherein the additive manufacturing process is one of laser/electron beam melting/sintering; powder bed fusion; 3D printing; selective laser sintering/melting; a blown powder process; direct laser deposition; freeform manufacture; or additive layer manufacturing.

5. The method according to Claim 1 or Claim 2 comprising forming the component body by machining.

6. The method according to any one of the preceding claims comprising forming the component body comprising one or more sacrificial portions which partly define the cavity.

7. The method according to Claim 5 further comprising removing the sacrificial portion(s) of the component body after the step of performing hot isostatic pressing.

8. The method according to any one of the preceding claims comprising forming the component body as a net or near-net shape component.

9. A consolidated component formed by the method according to any one of Claims 1 to 8.
